# EUROPEAN PATENT SPECIFICATION

(11) **EP 3 967 251 B1**
(45) Date of publication and mention of the grant of the patent: **14.02.2024**
(21) Application number: 20195864.2
(22) Date of filing: 12.09.2020
(51) Int. Cl.: A61B 17/86, A61B 17/88, B25B 15/02, F16B 25/00

(54) **TOOL ASSEMBLY FOR THE MANIPULATION OF SCREWS**
WERKZEUGANORDNUNG ZUR MANIPULATION VON SCHRAUBEN
ENSEMBLE OUTIL POUR LA MANIPULATION DE VIS

(43) Date of publication of application: 16.03.2022
(73) Proprietor: Flex Technology, Inc., Charlottesville, VA 22903 (US)
(72) Inventor: KRAUSE, William R, Charlottesville, VA 22902 (US)
(74) Representative: Verhees, Godefridus Josephus Maria

(56) References cited:
- US-A1- 2013 116 693
- US-A1- 2014 276 894

## Description

### Field of the Invention

The present invention pertains to a tool assembly for manipulating screws and, more particularly, to a tool assembly for manipulating flexible screws.

### BACKGROUND

The application of flexible fastening devices encompasses a broad spectrum of industries, included, but not limited to, manufacturing, construction, mining, transportation, agriculture, aviation, automotive, and medical. Flexible fastening devices, either tipped like screws or flat end like bolts, have the characteristics of the cylindrical portion of the device being bendable about the longitudinal length. Flexible fastening devices are useable in many applications, from manufacturing to medical, to secure two objects together.

In manufacturing and construction, fastening devices are used to join curved members together, to join misaligned holes, to absorb vibration between two components and numerous other applications. In addition, flexible devices are used to connect two or more members whereby a straight passage of the bolt is impossible and a curved passage in one member allows the inserted flexible screw or bolt to follow the passage and be joined to another member. For example, two curved tubes can be connected by inserting a flexible bolt through the internal diameter of one to thread into the internal diameter of the other tube to join them together.

In the medical industry, flexible screws are particularly useful in the intramedullary fixation of fractured or severed bone fragments. Bone screws are typically used in internal fixation to anchor the fixation system to the relevant bone portions or to join two or more fragments of a fractured bone into close proximity for bone union. For example, screws can be used in plate or rod systems to treat complex fractures of long bones or conditions such as vertebral instability. In small bone fractures, such as the bones of the hands, feet and wrist, the screw is placed across the fracture site to bring the fracture surfaces in close proximity. In medium (clavicle, rib and others) and long (lower and upper extremities) bone fractures, screws can be inserted into the intramedullary canal for minimally invasive fracture reduction.

Various surgical procedures utilize devices to fixate anatomical tissue for healing. An example of a fixation device is a compression bone fixation screw, commonly referred to throughout the present description as "compression screw", used to fixate two or more bone fragments or intramedullary fixation of a bone.

A surgical screwdriver is commonly used to insert bone screws. This form of screwdriver has a drive shaft for rotating the screw and advancing it along the longitudinal axis of the driver. The driver cooperatively engages with a drive recess, within the proximal end of the screw, to help achieve axial alignment of the screw with the drive shaft of the screwdriver. The screwdriver also has means of preventing rotation of the screwdriver with the guide wire over which the screw is placed.

A tool assembly according to the preamble of claim 1 is known from US2014/276894A1.

### SUMMARY OF THE INVENTION

A tool assembly for manipulating a fastening device (150), such as a bone screw, of at least one element has a flexible guide rod (120), a fastening device (150), and a handle sub-assembly (200).

The flexible guide rod (120) is comprised of a proximal threaded section (121), a mid-section (123), and a distal section (125) having an outwardly tapered distal end. The outward taper is about 1 degree for a Morse taper fit and less than 90 degrees for a non-Morse taper fit. The flexible guide rod (120) has at least one cross-section configuration and a length greater than that of the fastening device (150) to extend through the proximal receptacle (155) of the fastening device (150) and engage with the guide rod mating passage (247) of the handle sub-assembly. The material for the flexible guide rod can be selected from the group consisting of Nitinol, spring steel, flexible polymers and flexible composites.

The fastening device has a distal segment (156) with an open tapered distal end (162); a proximal segment (154) with an open proximal end (164); and a central flexible segment (152) extending from the distal segment (156) to the proximal segment (154). The distal segment (156) and proximal segment (154) are preferably threaded.

The central flexible segment (152) has at least one sinuous slot running its length to provide flexibility and enable the connection of non-linear elements. A fastener channel (167), having at least one cross-section configuration, extends from the tapered distal end (162) of the distal segment (156) to the proximal receptacle (155) at the open proximal end 164. The cross-section configuration of the fastener channel (167) is complimentary to at least one of the cross-section configurations of the flexible guide rod (120). The tapered internal surface (166) of the distal end (156) of the fastening device (150) is tapered to the same degree as the distal tapered section (125) to enable the two elements to engage with one another. The tapered internal surface (166) of the fastening device (150) and the distal section (125) of the guide rod (120) are dimensioned to prevent the distal end (128) of the distal section (125) from passing through the distal end (162) of the fastening device (150). The cross-section of the distal alignment surface (159) of the fastening device (150) is complimentary to the predetermined configuration of the guide rod 120 to enable rotation.

A handle sub-assembly (200) has a handle (210) with a clamp orifice (212) extending into the handle (210) from a first end past a mid-point of the handle (210). A handle shank receiving area (211) extends through the handle (210) from the top to a handle shank (220) that extends from the bottom of the handle (210) from the bottomside of the handle (210). A coupling mechanism (230) extends from the handle shank (220) that is configured to receive proximal receptacle (155). A guide rod channel (228) that extends through the coupling mechanism (230) is configured to receive the proximal end of the flexible guide rod (120). The exterior of the coupling mechanism (230) has a cross section complementary to a cross section of a proximal mating surface (153) of the open proximal end (164) of the fastening device (150).

A guide rod shank passage (222) extends from the guide rod channel (228), through the handle shank (220) to the handle shank receiving area (211).

A guide rod holder clamp (250) having a securing member receiving area is dimensioned to be received within the clamp orifice (212). A guide rod hole (254) with the guide rod holder clamp (250) is dimensioned to align with the guide rod shank passage (222) and receive the flexible guide rod (120). A guide rod holder clamp securing member is configured to interact with the securing member receiving area. The securing member receiving area of the guide rod holder clamp (250) is optimally comprised of interior threads (252) and the tightening member a guide rod holder clamp screw (255) having threads (257) to interact with the interior threads (252).

A guide rod holder assembly (238) comprises a guide rod holder (240) and holder shaft (244). A holder shaft (244) has a guide rod mating passage (247) extending through a distal portion of the holder shaft (244); and a guide rod shaft passage (245) extending through a proximal portion of the holder shaft (244). The guide rod holder (240) and the guide rod shaft passage (245) are dimensioned to receive the flexible drive rod (120). The guide rod passage (245) is generally circular. The guide rod mating passage (247) has a cross-section complimentary to that of the guide rod (120) to enable rotation of the guide rod (120) by the guide rod holder (240).

One of the at least one cross-section configuration of the flexible guide rod is configured to interact with at least one of the at least one cross-section configuration of the fastener channel 167 to enable the fastening device to be inserted into or removed from at least one element. The guide rod (120) has a cross section less than an interior cross section of the distal segment (156) to create a gap (142) for torque transmission while enabling manipulation of the fastening device (150). The guide rod (120) cross section is less than the guide rod shaft passage (245) of the guide rod shaft (244) to create a gap (148) for torque transmission while enabling manipulation of the fastening device (150).

A locking nut, having interior threads (136) dimensioned to be received on the guide rod (120) proximal threaded section (121) and a body (130) dimensioned to be received within the proximal receptacle (155) of the fastening deice (150) is used to tighten pull the end of the screw toward the proximal end. This prevents movement by compressing the sinuous slot.

The steps for assembling the tool assembly for the manipulation of a fastening device are:
a. Inserting a guide rod holder clamp (250) having a guide rod hole (254) and a threaded receiving area into a clamp orifice (212) within a handle (210) of a handle sub-assembly (200);
b. Aligning said guide rod hole (254) with a holder shaft receiving area (211) within handle (210);
c. extending a holder shaft (244) of a guide rod assembly (238) through said holder shaft receiving area (211) and into a handle shank (220) extending from a bottom side of said handle (210) to a coupling mechanism (230);
d. Inserting a proximal threaded section (121) of a flexible guide rod (120), having at least one cross-sectional configuration, into an open tapered distal end (162) of a fastening device (150) having a fastener channel (167) with a cross-sectional configuration;
e. extending said flexible guide rod threaded section (121) through a distal segment (156) and a central flexible segment (152) to extend beyond a proximal segment (154) of said fastening device (150);
f. Continuing to insert said flexible guide rod (120) through said fastener channel (167) until a tapered distal end (128) of a distal section (125) of said guide rod (120) contacts said open tapered distal end (162) of said fastening device (150);
g. inserting said threaded section (121) of said flexible guide rod (120) into a guide rod channel (228) within said coupling mechanism (230);
h. inserting said coupling mechanism (230) into a proximal receptacle (155) within a proximal segment (154) of said fastening device (150);
i. aligning a cross-section configuration of said proximal receptacle (155) with a cross section configuration of said coupling mechanism (230);
j. aligning a cross section configuration of said threaded section (121) with a cross section configuration of said guide rod shaft passage (245) of holder shaft (244);
k. preventing movement of said holder shaft (244) by pulling said guide rod hole 254 against said holder shaft 244 by inserting a threaded securing member into said guide rod holder clamp (250);
l. manipulating said fastening device (150) by rotating said guide rod holder (240) within said guide rod assembly (238) to engage tapered distal section 125 of guide rod 120 with fastener channel 167 at said distal segment (156) of said fastening device (150) to initiate rotation of said of said fastening device (150) at said tapered distal end (128);
m. removing said handle sub-assembly (200) when said fastening device (150) is positioned;
n. threading a nut (130) onto said threaded section (121);
o. pulling said tapered distal section (125) into said tapered distal end (162) of said fastening device (150) by tightening said nut (130); and
p. removing excess threaded section (121).

### Brief Description of the Drawings.

The objects, features, advantages and aspects of the present invention can be better understood with reference to the following detailed description of the preferred embodiments when read in conjunction with the appended drawing figures. All of the figures are drawn on an oversized scale, and like structure in different figures bears like reference numerals.
Figure 1 is a perspective view of the tool assembly 100 with screw 150 and attached handle sub-assembly 200 in accordance with the disclosed invention;
Figure 2 is an isometric view of a screw 150 and guide rod sub-assembly 110 separated from the handle sub-assembly 200 in accordance with the invention;
Figure 3 illustrates a view of the flexible guide rod 120 of the guide rod sub-assembly of Figure 2 in accordance with the invention;
Figures 4 A -E illustrates a close and sectional views of flexible guide rod 120 in Figure 3 in accordance with the invention;

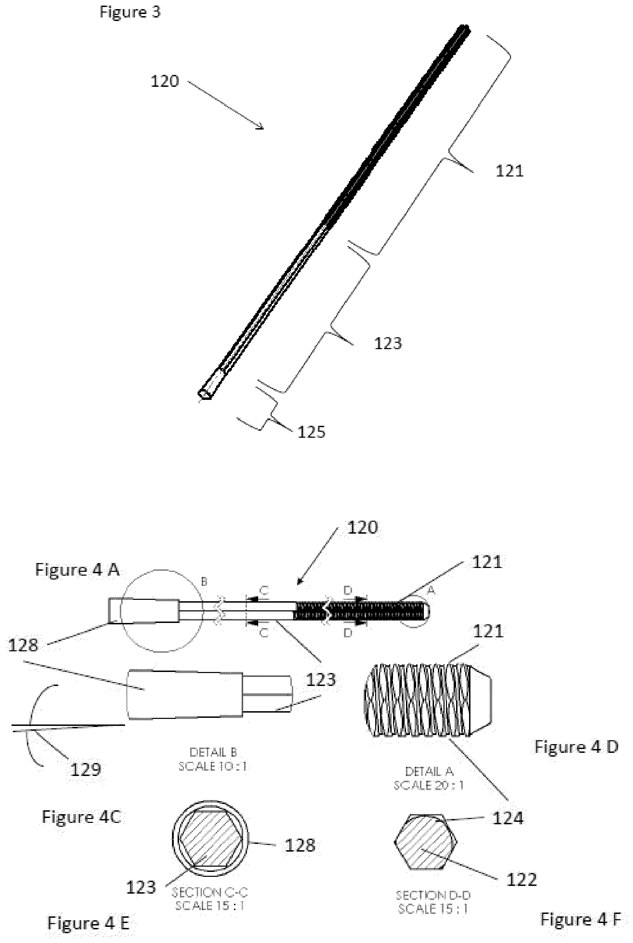

Figure 4 A is a longitudinal view of the flexible guide rod 120 with a breakaway cut and showing locations of the detail views A and B and sectional views D and E;
Figure 4 B shows the distal end 128 of the flexible guide rod 120 with an outward taper angle 129;
Figure 4 C shows taper angle 129 of the distal end of the guide rod 120;
Figure 4 D shows the fine threads 121 on the uniquely shaped guide rod threaded portion 122 at the proximal end of the guide rod 120;
Figure 4 E shows the cross-sectional shape in the unthreaded mid-section 123 of the guide rod 120 at section C-C;
Figure 4 F shows the cross-sectional shape of the proximal threaded section 122 of the guide rod 120 at section D-D;

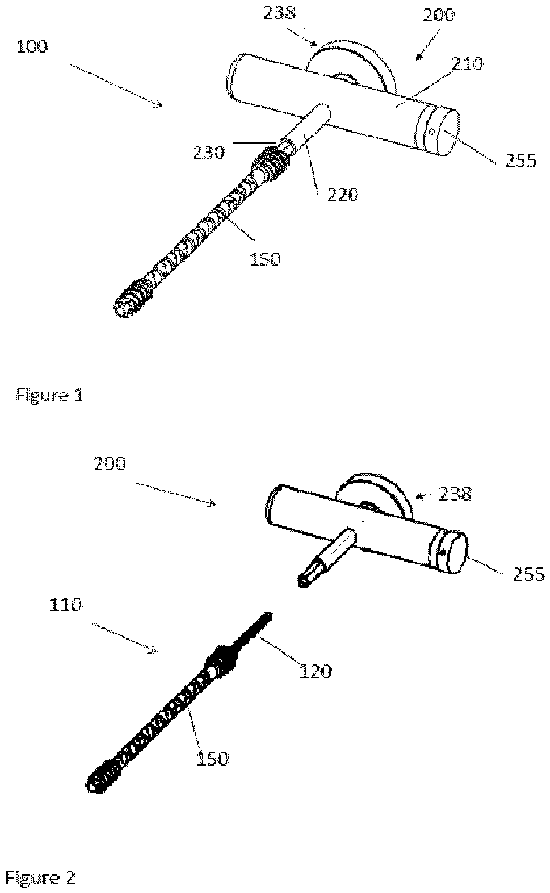

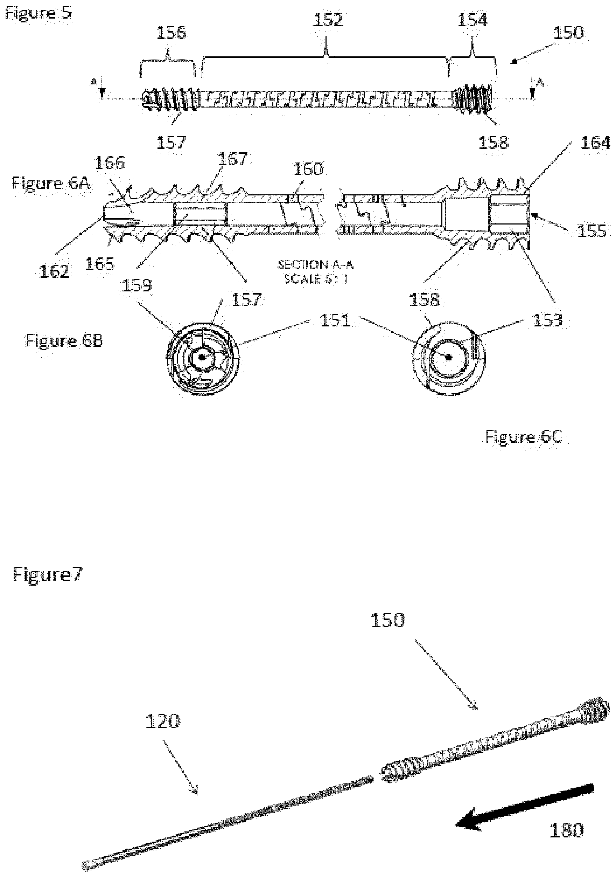

Figure 5 shows a fastening device 150 with its primary regions of the type applicable for medical use with the tool assembly of Figure 1 in accordance with the disclosed invention;
Figure 6A is a section view along plane A of the fastening device 150 of Figure 5 in accordance with the invention;
Figure 6B is a cross-sectional view of the threaded distal section 159 of Figure 6A in accordance with the invention;
Figure 6C is a cross-sectional view of the proximal section 158 of Figure 6A in accordance with the invention;
Figure 7 illustrates the fastening device 150 prior to sliding over guide rod 120;

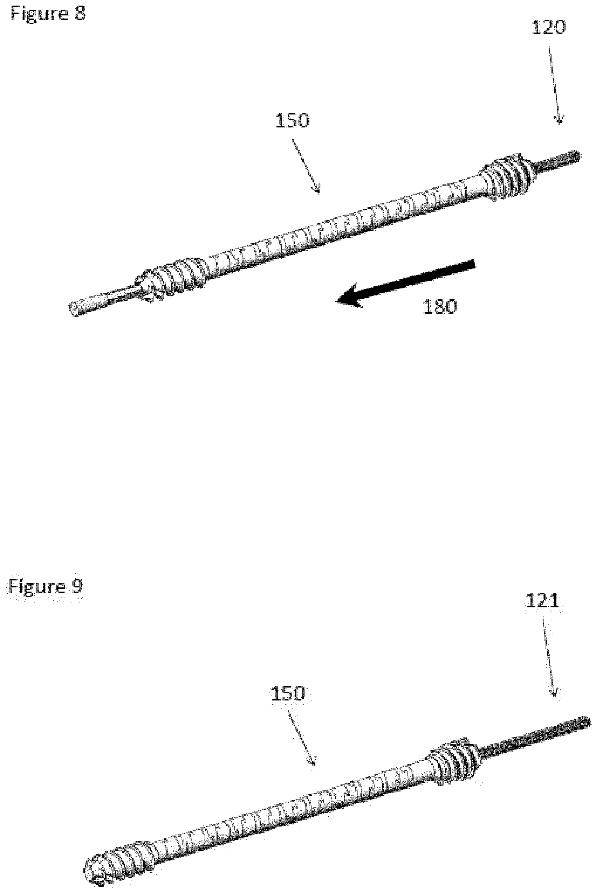

Figure 8 illustrates the fastening device 150 sliding over guide rod 120;
Figure 9 illustrates the fastening device 150 fully engaged with the guide rod 120;

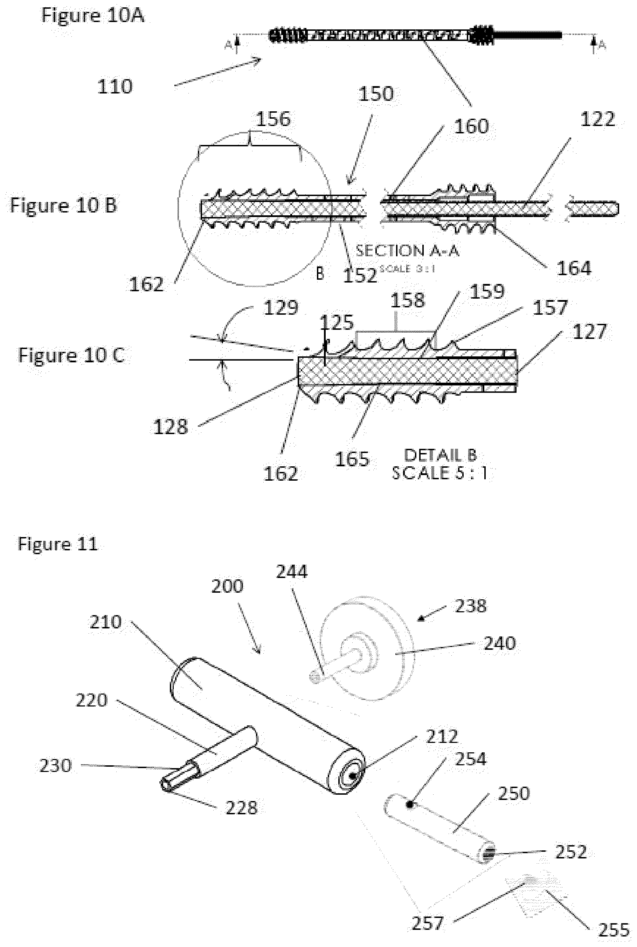

Figure 10A is a side view of the fastening device 150 fully engaged with the guide rod 120 and the location of Section A-A in accordance with the invention;
Figure 10B is a cutaway view of Section A-A of Figure 10A;
Figure 10C is a detailed cutaway view of Section B of Figure 10B;
Figure 11 shows an exploded view of the handle sub-assembly 200;

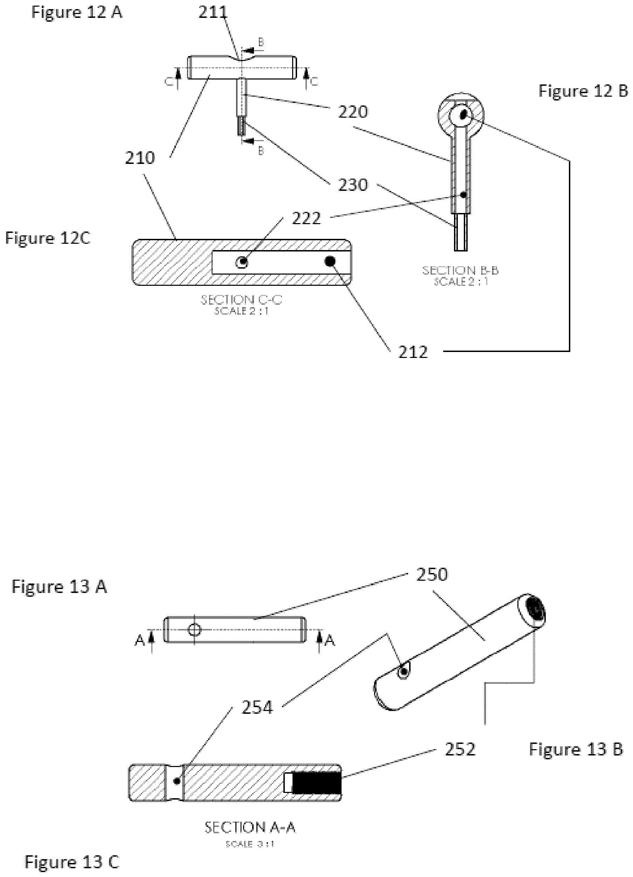

Figure 12A shows the handle sub-assembly 200 for the location of the section lines B-B and C-C;
Figure 12B is the detailed view of Section B-B in Figure 12A;
Figure 12C is the detailed view of Section C-C in Figure 12A;
Figure 13A is a top view of guide rod holder clamp 250 and location of Section A-A;
Figure 13 B is an isometric view of the guide rod holder clamp 250;
Figure 13C is the sectional view A-A of the guide rod holder clamp 250;

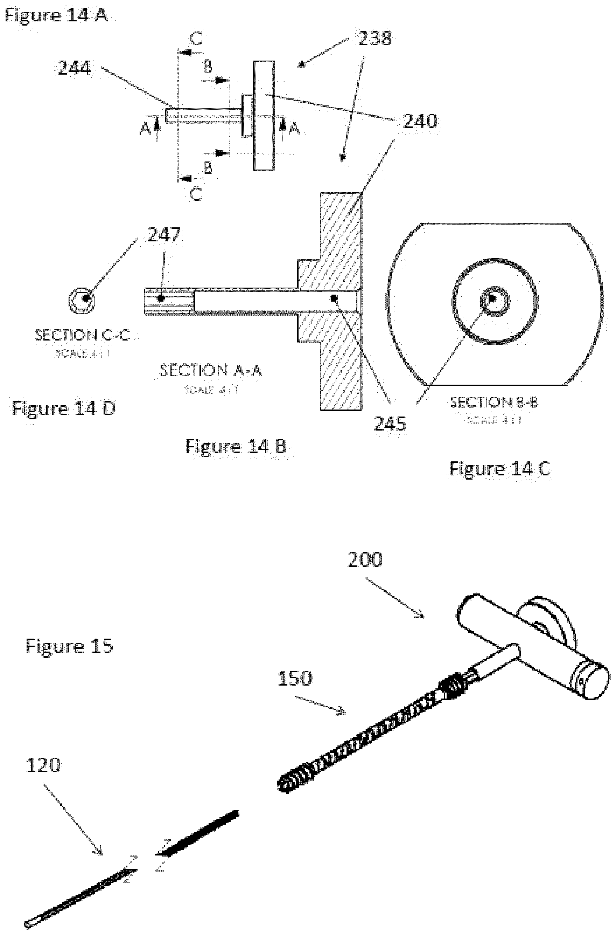

Figure 14A is a side view of the guide rod assembly 238 showing the guide rod holder 240, holder shaft 244, and location of Section A-A;
Figure 14B is the sectional view A-A of the guide rod holder 240;
Figure 14C is a sectional view of B-B of Figure 14A;
Figure 14D is a sectional view of C-C of Figure 14A;
Figure 15 shows the configuration of the handle sub-assembly 200 with attached fastening device 150 prior to inserting over the guide rod 120;

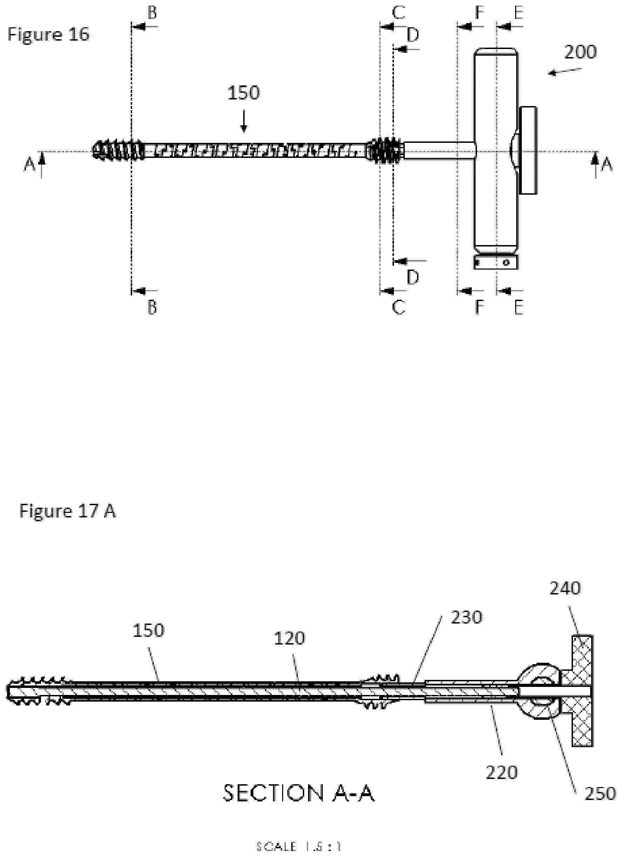

Figure 16 shows a side view of the assembled tool assembly 100 and the locations for the sectional views in Figures 17A-G;
Figure 17A shows the longitudinal section A-A of Figure 16;

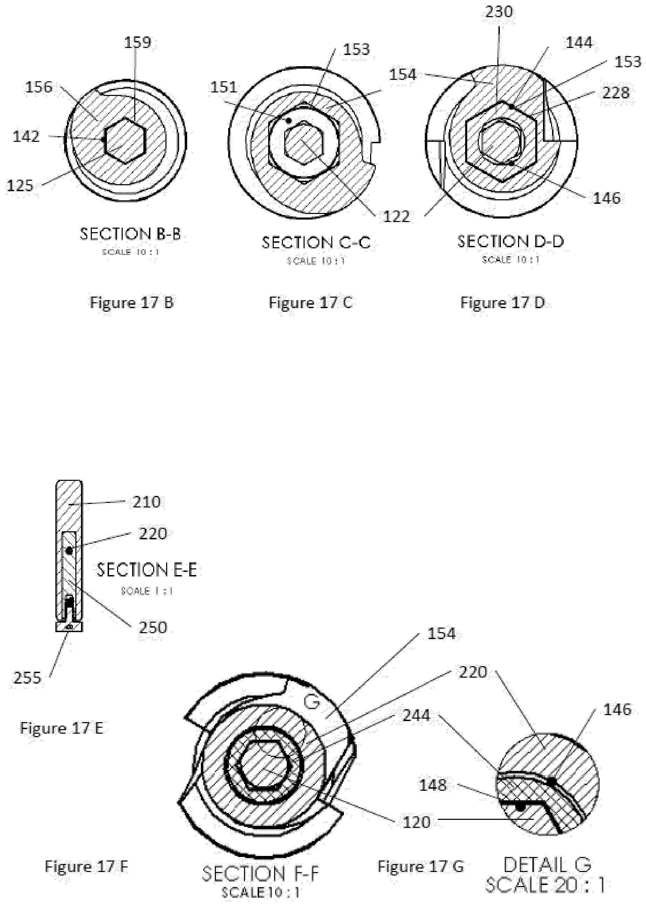

Figure 17B shows a cross sectional view B-B through the distal segment 156 of the fastening device 150 and the distal tapered end section 125 of guide rod 120 of Figure 16;
Figure 17C shows the cross-sectional view C-C through the distal portion of the proximal segment 154 of Figure 16;
Figure 17D shows the cross-sectional view C-C through the proximal portion of the proximal segment 154 of Figure 16;
Figure 17E is the sectional view E-E of the insertion tool handle 210 of Figure 16;
Figure 17F shows the cross-sectional view F-F through the insertion tool handle shank 220 of Figure 16;
Figure 17G is the detail area G-G shown in Figure 17F;

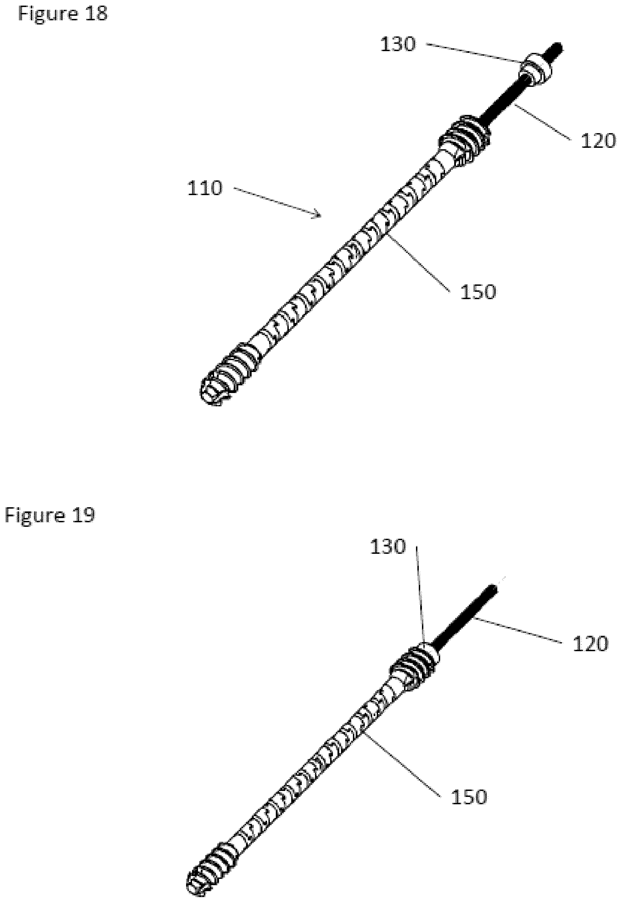

Figure 18 shows the guide rod sub-assembly 110 with the associated locking nut 130 on the guide rod 120;
Figure 19 show the guide rod sub-assembly 110 with the associated locking nut 130 fully on the guide rod 120;

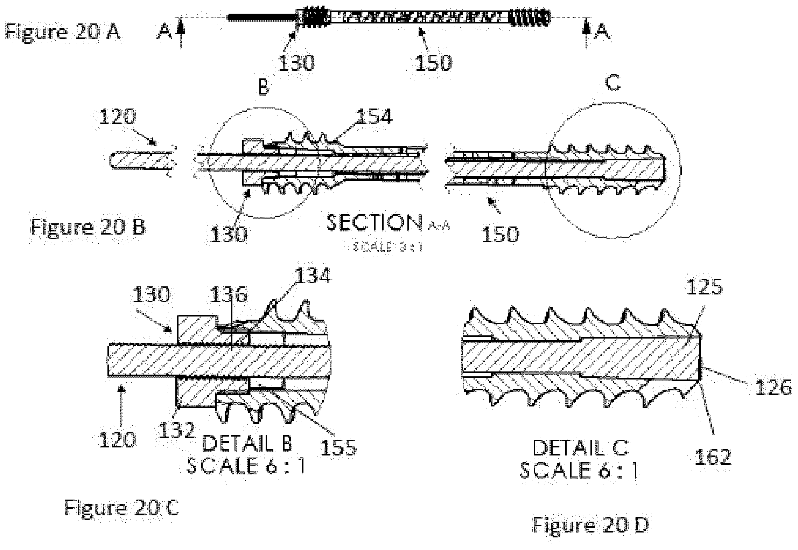

Figure 20A shows a lateral view of the locking nut 130 fully on the guide rod 120 and the location of Section A-A;
Figure 20B shows the cross-section A-A and the location of detail areas B and C;
Figure 20C shows the detail area B with the locking nut 130 fully engaged
Figure 20D shows the detail area C with the taper end 128 fully engaged with the screw 150;

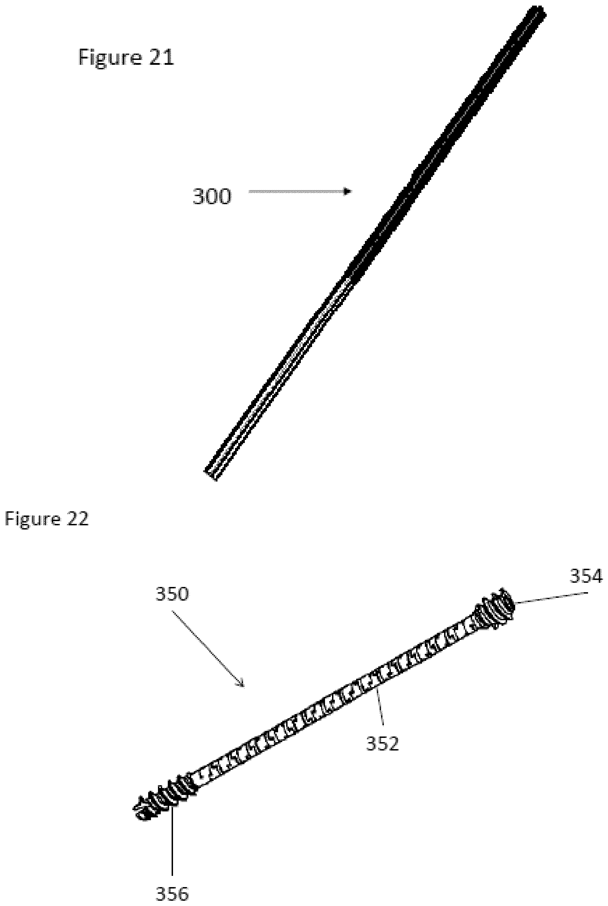

Figure 21 is an alternate embodiment of a guide rod 300; and
Figure 22 is an alternate embodiment of a fastening device 350.

### Detailed Description of the Invention

### Definitions

As used herein the term "about" shall refer to plus or minus ten percent (10%).

As used herein the terms "fastening device", "screw", and "bolt" shall be used interchangeably and reference any flexible device that can secure two or more objects together.

As used herein the term "manipulation" or "manipulating" shall refer to inserting, removing, tightening, loosening, or adjusting of a screw; the terms "manipulation" or "manipulating" can be used interchangeably with "drive" or "driving" or "driven".

As used herein the term "compression fastening device" shall refer to any flexible device that can secure two or more objects together and tighten the contact through causing the fastening device to shorten.

As used herein the term "complimentary configuration" will mean shapes of interacting parts that interact with one another to prevent rotation of interacting parts individually.

As used herein the term "distal" shall refer to situation closer to the point of interaction between the tool assembly and an intended element.

As used herein the term "proximal" shall refer to situation farther away from the point of interaction between the tool assembly and an intended element.

As used herein the term "element" shall refer to any object or objects requiring the manipulation of a screw.

The use of flexible fasteners, such as bone screws, is hindered by the twisting of the screw when manipulated by the proximal end. In addition, broken screws within an element are also a problem and a mechanism or tool is needed to remove the distal portion of a broken screw.

It has been determined that in a screw having an interior cavity or channel, if the interior cavity of the distal end of a screw has a shape that is complimentary to the shape of a rod that can be inserted into the cavity from the proximal end, the screw can then be driven from the distal end. In this way, the screw can be inserted, removed, tightened, loosened, or otherwise adjusted as needed by application of force at the distal end of the screw. This is especially applicable when the screw is flexible and can be in a curved orientation, such as a bone cavity, through which the rod must pass to enter the distal end of the screw. The ideal choice of material for the guide rod portion of this application is Nitinol, a nickel- titanium alloy, although other metallic alloys or flexible polymers and composites meeting the criteria set forth herein are applicable.

Removal of a screw from the distal end also facilitates removal when the screw is broken. When inserted into the distal cavity, the complimentary rod can be used to rotate the screw in the opposite direction from insert, resulting in the screw backing itself out. Although it is common to back out a non-flexible screw, flexibility and screw length have prevented this from being easily accomplished heretofore.

Similarly, the rod can also be used to insert the screw. Thus, when coupled with a driving attachment to the proximal end of the screw, the driver would be applying the driving torque to both the distal and proximal ends of the screw thus relieving the twisting of the central flexible segment of the screw. For exemplary purposes, the insertion rod and internal complimentary shaped internal cavity are shown as hexagonal, but any complimentary shapes that do not allow rotation relative to one another are applicable.

Further, in prior art devices the alignment of the guide rod with the distal internal mating cavity and the mating cavity on the handle was dependent upon the alignment of the internal driving cavity at the distal end of the screw which receives the complimentary shaped driving coupling mechanism on the driver. This problem is eliminated in the disclosed system by having a separate element that is complimentary to the shape of the guide rod and is initially allowed to rotate freely but locks in place for driving the screw when necessary. In addition, other improvements have been incorporated into the design to ease manufacturing.

The disclosed driver couples at both the proximal and distal ends of the intended screw with following characteristics.

The embodiment illustrated in Figure 1 shows the tool assembly 100 illustrating the handle sub-assembly 200 and attached fastening device 150 in accordance with the disclosed invention for the manipulation of screws such as insertion, removal, tightening, loosening, or other adjustment. The handle sub-assembly 200 enables the manipulation of both flexible, as well as inflexible, screws. The handle sub-assembly 200 comprises a handle 210, a handle shank 220, a coupling mechanism 230, guide rod holder clamp screw 255, and a guide rod holder assembly 238. The coupling mechanism 230 has an external configuration to interact with the proximal end of the intended fastening device 150. The flexible guide rod 120 penetrates the fastening device's 150 internal opening to couple with the device's internal distal end through complimentary tapered regions as described in more detail hereinafter.

As illustrated in Figure 2, the guide rod sub-assembly 110 is composed of the guide rod 120 and fastening device 150 that are attached to the handle sub-assembly 200. When attached, guide rod 120 is secured within the handle sub-assembly 200 by the guide rod holder assembly 238 while fastening device 150 is secured to the handle sub-assembly 200 by coupling mechanism 230. The coupling mechanism 230 has an external configuration to interact with the proximal end of the intended fastening device 150.

Guide rod 120 is comprised of three sections as illustrated in FIGS. 3 and 4A - F. The guide rod 120 typically has a unique cross-sectional shape that is complimentary to the distal alignment surface 159 of the fastening device 150. The guide rod 120 cross-sectional shape is shown as a hexagonal in Figures 4D and 4E. A hexagon is the preferred shape, but other shapes could provide the same functional results. The interior shape of the distal alignment surface 159 is complimentary to the guide rod's shape and sized to prevent excessive rotation between the two. As the tapered end 128 is not used to rotate the fastening device 150, the cross-sectional shape is preferably round for ease of manufacturing, however other shapes can be used. The rod 120 consists of the distal tapered end section 125, the mid-section 123, and the proximal threaded section 121. The flexible guide rod 120 penetrates the internal cavity of fastening device 150 wherein distal tapered section 125 couples with the internal distal end of fastening device 150 through complimentary tapered regions as described in more detail hereinafter. The proximal threaded section 121 is better illustrated in Figures 4A, 4C and 4E.

Figure 4A provides a cross-sectional view of the guide rod 120 as well as identification of the areas illustrated in detail in subsequent figures, including the distal tapered end 128. Figure 4D is a more detailed view of the fine threads 122 of the guide rod proximal threaded portion 121 of the guide rod 120. As shown in Figure 4B, the distal tapered end section 125 has an outward taper towards the distal tapered end 128 with a taper angle 129 from about 1 degree for a Morse taper fit, to less than about 90 degrees for a non-Morse taper fit.

The mid-section 123 has a unique cross-sectional shape, as shown in Figure 4E, that is complimentary to the internal cavities of the fastening device 150 and the guide rod sub-assembly 238. For convenience, the guide rod 120 has a hexagonal shape as shown in the illustrations but could be one of numerous shapes that will prevent rotation between the fastening device 150 and the guide rod 120 and the guide rod holder 240 of the guide rod sub-assembly 238. Figure 4F illustrates the section D-D through the threaded portion 121 of guide rod 120 showing the cross section of the rod 122 with the external threads 124. Due to the small diameter of the guide rod 120, about 1 to 5 mm, the guide rod holder 240 will have a small unique shaped interior cavity 245 (Figure 14B).

Figure 5 illustrates the fastening device 150 with its proximal segment 154 containing proximal threads 158, distal segment 156 with distal threads 157, and central flexible segment 152 therebetween. The cross-sectional view of Figure 6A illustrates the proximal receptacle 155 for receiving the fastener coupling mechanism 230 of the handle sub-assembly 200 that is illustrated in detail in Figure 11. For the sake of clarity and convenience, the proximal mating surface 153 of the proximal receptacle 155 is depicted as a hexagonal shape to receive a hexagonally shaped fastener coupling mechanism 230 on the handle sub-assembly 200. Preferably the proximal receptacle 155 and fastener coupling mechanism 230 are of the appropriate shape and complimentary to ensure an adequate transmission of torque for the fastening device 150 insertion or withdrawal. As with the proximal receptacle 155 and fastener coupling mechanism 230 requiring complimentary configurations, the distal alignment surface 159 and the unthreaded portion 127 of the guide rod 120 require complimentary configurations.

Figure 6A also illustrates clearly the tapered internal surface 166 of the distal tapered exterior surface 165 of the fastening device 150. The tapered internal surface 166 has a taper complimentary to that of the tapered end 128 of the guide rod 120, thereby dimensioning the tapered end 128 to fit within the tapered internal surface 166. As the taper is wider at the distal end 162 of the fastening device 150 as well as the tapered end 128 of the guide rod 128, the complimentary taper will prevent the dislodging of the guide rod 120 during application of the locking nut 130 as described in more detail in Figure 19. Also illustrated in this figure are the proximal end 164 of the proximal segment 154 and the helical slots 160 within the central flexible segment 152 of the fastening device 150.

Figures 6B and 6C show the end views of the fastening device 150 and its unique distal alignment surface 159 within the proximal receptacle 155 As illustrated in Figure 6B the distal alignment surface 159 of the distal segment 156 is such that it mirrors the cross-sectional shape of the mid-section 123 of the guide rod 120.

The interior shape of the proximal segment 153 mirrors the exterior shape of the fastener coupling mechanism 230 of the handle sub-assembly 200.

Figure 7 show the fastening device 150 about to be slide over the guide rod 120 during the course of insertion as shown in the direction of the arrow 180.

Figure 8 shows the fastening device 150 slid part way over the guide rod 120 during the course of insertion as shown in the direction of the arrow 180.

Figure 9 shows the fastening device 150 slid all the way over the guide rod 120 during the course of insertion. The proximal threaded section 121 of the guide rod 120 extends beyond the fastening device 150 a sufficient distance to extend through the handle assembly 200 and into the guide rod assembly 238. The length of the guide rod 120 needs to pass into the guide rod assembly 238 a sufficient distance to be engaged for rotation. This facilitates manufacturing in that the guide rods 120 have the option be manufactured in approximate sizes, e.g. short, medium, and long rather than requiring exact sizing.

Figure 10A shows the screw-guide rod assembly 110 and the location for Section A-A shown in Figure 10B.

Figure 10B shows Section view A-A with the location for Detail B. Shown is the fastening device 150 positioned over the guide rod 120. The guide rod 120 extends from the distal end 162 of the fastening device 150, though the central flexible segment 152 created by helical slots 160 and out through the open proximal end 164. The proximal threaded portion 122 of the guide rod 120 extends beyond the proximal end 164 of the fastening device 150 for passage and interaction with the insertion handle sub assembly 200.

Figure 10C shows the Detail B of Figure 10B and the distal tapered end section 125 and tapered end 128 of the guide rod 120 within the distal segment 156 of the fastening device 150. The taper angle 129 is such that the distal tapered end section 125 mates with the surface of the distal tapered surface 165 of the fastening device 150.The alignment between the tapered end 128 and the distal end 162 is also clearly shown in this figure. Typically, it is preferred that the taper angle 129 is that recommended for a Morse Taper in the order of 1 degree. During final installation, this will allow the distal tapered end section 125 of the guide rod 120 to be firmly joined to the distal tapered surface 165 of the fastening device 150 by means of a friction fit.

Figure 11 illustrates an exploded view of the handle sub-assembly 200 and the component parts of the sub-assembly 200. The handle 210 has, at one end, a clamp orifice 212 that extends into the handle 210 past the guide rod shank passage 222 inside of handle shank 220 (Figure 12B). The guide rod holder clamp 250 has a hole 254 which, when in place, aligns with the guide rod shank passage 222, illustrated more clearly in Figures 12 A - 12C. The guide rod shank passage 222 is an extension of the guide rod channel 228 within the fastener coupling mechanism 230 and, in combination, they enable the guide rod 120 proximal threaded section 121 to interact with the complimentary interior of the guide rod mating passage 247 (Figure 14A and Figure 14D).

For use, the guide rod holder clamp 250 is inserted in clamp orifice 212 in the handle 210 such that the guide rod hole 254 is aligned with the guide rod shank passage 222 shown in Figure 12B of the handle shank 220. This alignment is necessary so that the guide rod holder shaft 244 can be inserted into the guide rod shank passage 222. The clamp screw threads 257 of the guide rod holder clamp screw 255 are inserted into the internal threads 252 of the guide rod holder clamp 250. The guide rod holder clamp screw 255 is used to secure the guide rod holder 240 and handle shank 244 when it is inserted through the guide rod holder 240 within the insertion handle 210. Although the use of a guide rod holder clamp screw 255 engaging with the threads 252 within the guide rod holder clamp 250 is an easy to manufacture method of preventing movement of the guide rod 120, other methods as known in the art can be used.

It is critical during manufacture that the guide rod shank passage 222 and the guide rod hole 254 are aligned so that once the guide rod is inserted, the threading of the guide rod holder clamp screw 255 locks the holder shaft 244 in place.

Figure 12 A illustrates the orientation of the sectional views in the front view of the handle sub-assembly 200 shown in Figures 12B and 12C for detail of the internal cavities of the part. The holder shaft 244 receiving area 211 is also illustrated in this figure. The holder shaft receiving area 211 is dimensioned to receive the holder shaft 244 in a manner to allow rotation and alignment without excessive side to side movement.

Figure 12B shows the vertical section, Section B-B with the guide rod shank passage 222 running through the handle 210 and the length of the handle shank 220 and the channel within the fastener coupling mechanism 230 of the handle sub-assembly 200 for receiving the guide rod holder shaft 244 and the guide rod 120.

Figure 12C shows the horizontal section, Section C-C of the handle 210 of Figure 12A and the location and orientation of the guide rod shank passage 222 and clamp orifice 212 for the guide rod holder clamp 250.

Figures 13 A, 13B and 13C illustrate the top, isometric and longitudinal section view, respectively, of the guide rod holder clamp 250. The positioning of the internal threads 252 that engage the guide rod holder clamp screw 255 are clearly illustrated in this figure.

Figure 14A illustrates the side view of the guide rod assembly 238, comprising the guide rod holder 240, holder shaft 244, and the locations of sectional views A-A, B-B and C-C. Longitudinal section A-A is shown in Figure 14B illustrating the guide rod shaft passage 245 within holder shaft 244 for the passage of the guide rod 120 through the guide rod holder 240. The interior shape of the holder shaft 244 and thus the shape of guide rod shaft passage 245 is primarily circular as shown in Figure 14C of Section B-B to provide unimpeded passage of the guide rod 120. However, the guide rod mating passage 247 of the distal end, or other section within the shaft, requires a shape complimentary to the cross sectional shape of the guide rod 120 as shown in section C-C of Figure 14D. Guide rod mating passage 247 provides rotational control and force to the guide rod 120 when it is inserted in the guide rod holder 240 and locked in the handle sub assembly 200. The shape of the guide rod mating passage 247 is illustrated as a hexagonal for convenience and can be any configuration complimentary to the guide rod 120.

In Figure 15, the fastening device 150 has been inserted on the handle sub-assembly 200 and is slid on the guide rod 120 (shown with in break way for clarity). The unique cross-sectional shape of the guide rod 120 conforms to the complimentary internal guide shape of the distal tapered surface 165 of fastening device. As well as the internal guide shape of the guide rod mating passage 247 of the guide rod holder 240.

With the fastening device 150 fully inserted over the guide rod 120, the relationships of the parts can be examined in the sectional views presented in Figure 16. During insertion the fastening device 150 is driven in contributing locations depicted at Section B-B, Section D-D, Section F-F and Section E-E

Section A-A of Figure 16 is shown in Figure 17A showing the guide rod 120 fully through the length of the fastening device 150, passing through the fastener coupling mechanism 230 within the handle shank 220, and positioned within the guide rod holder 240. The guide rod 120 is maintained within the guide rod holder 240 by the guide rod holder clamp 250.

The cross-section B-B in Figure 17B shows the complimentary relationship between the fastening device's threaded section 157 of the distal segment 156 and the distal tapered end section 125 of the guide rod 120. When the insertion torque is transmitted through the guide rod 120 to the distal tapered end 128, the guide rod rotates and interacts with the complimentary interior of the distal segment 156 of the fastening device 150 along the distal alignment surface 159. The distal gap 142 between the distal alignment surface 159 and the guide rod unthreaded surface 127 must sufficiently small to provide torque transmission but not to allow the guide rod 120 to rotate freely.

Figure 17C shows the cross section at Section C-C in Figure 17A with the guide rod threaded portion 122 within the distal section of the proximal segment 154, proximal mating surface 153, and the proximal receptacle 155.

Figure 17D shows the cross section at Section D-D in Figure 17A with the guide rod threaded portion 122 within the proximal section of the proximal segment 154. The fastening coupling mechanism 230 of the handle sub-assembly 200 is shown within the proximal segment 154 where the proximal mating surface 153 and proximal screw coupling 228 interact for insertion through the proximal gap 144. Also illustrated is the gap 146 between the handle shank 220 and the holder shaft 244.

Figure 17E illustrates the Section E-E of the insertion tool handle 210 and the mechanism to lock the handle shank 220 within the guide rod holder clamp 250. When the guide rod holder clamp screw 255 is rotated into the guide rod holder clamp 250, the guide rod holder clamp screw 255 is drawn outward and the handle shank 220 is unable to rotate and held in place to transmit torque to the guide rod 120 captured within the guide rod holder clamp 250 and to the distal end of the fastening device 150.

Figure 17F illustrates the cross-section F-F in Figure 17A, through the handle shank 220 of the handle sub-assembly 200. The cross-section of the guide rod 120 is complimentary to the guide rod mating passage 247 (Figure 14B) of the guide rod holder 240 to transmit the insertion or removal torque during installation once locked in place with the guide rod holder clamp 250. Prior to clamping, the holder shaft 244 of the guide rod holder 240 is free to rotate within the handle shank 220 to allow for the alignment of complimentary configurations between the guide rod 120 and the distal alignment surface 159 of the fastening device 150. Immediate locking of the holder shaft 244 within the handle shank 220 would make aligning the configurations difficult. Once aligned, the placement is locked into place through the tightening of the guide rod holder clamp screw 255.

Figure 17G provides a detailed view of the gap 146 and gap 148 between the guide rod 120 and the guide rod holder shaft 244. The circular configurations of the interior of the handle shank 220 and the exterior of the holder shaft 244 are dimensioned to form a gap 146 to permit rotation between the two parts. The hexagonal configuration between the guide rod 120 and the interior of the holder shaft 244 enables the gap 148 to prevent rotation between the two parts and allows the fastening device 150 to be rotated for manipulation. The size of the gaps 146 and 148 is dependent upon the size (diameter) of the fastening device 150 and the guide rod being used and will be known to those versed in the mechanical arts.

With the fastening device 150 fully placed and engage, the handle sub-assembly 200 is disassembled and removed from attachment to the fastening device 150. The guide rod locking nut 130 is then threaded onto the threaded portion 122 of the guide rod 120, as illustrated in Figures 18 and 19

The guide rod locking nut 130 is threaded down to the proximal end 164 of the fastening device 150. This draws the tapered end 128 of the guide rod 120 against the tapered distal end 162 of the fastening device 150 to compress and stiffen the fastening device 150. Once firmly in place the guide rod 120 is cut off proximal to the guide rod locking nut 130.

Figure 20A shows the guide rod nut 130 fully engaged on the guide rod 120 and the location of Section A-A; As can be seen in Figures 20B and 20C, the head 132 of the locking nut 130 is in contact with the edge of the proximal segment 154 of the fastening device 150. The body 134 of the locking nut 130 extends into the proximal segment 154 to provide sufficient contact with the threads 124 of proximal threaded section 121 of the guide rod 120. The threads 136 of the locking nut 130 engage with the threads 124 of the guide rod 120, pulling the distal tapered end section 125 into the distal tapered internal surface 166 of the fastening device 150. Once tightened, the distal edge 126 of the distal tapered end section 125 is flush with the distal end 162 of the fastening device 150. As previously stated, the taper of the distal tapered end section 125 prevents the guide rod 120 from slipping. As the guide rod 120 pulls the distal end 162 of fastening device 150 toward the proximal section 121 of the guide rod 120, the helical slot is tightened, forcing the teeth to interlock in position.

In preparation for use the guide rod holder clamp 250 is placed in the clamp orifice 212 with the guide rod hole 254 positioned to receive the holder shaft 244 of the guide rod assembly 238. The holder shaft 244 is placed in the handle 210, through the guide rod hole 254 and into the holder shank 220. The proximal threaded section 121 of the guide rod 120 is slid first through the distal segment 156 of the fastening device 150 until the distal tapered end section 125 comes in contact with the distal end 162. The proximal threaded section 121 is then run though the guide rod channel 228 to engage with the guide rod mating surface 247. The complimentary configurations between the guide rod mating surface 247 and the distal alignment surface 159, once aligned, enable coordinated rotational movement between the guide rod 120 and the guide rod assembly 238. Once alignment is achieved, the guide rod holder clamp screw 255 is tightened with in guide rod holder clamp 250, applying pressure to the threaded section 121 and locking the guide rod 120 in position. Once the fastening device 150 is in position the handle sub-assembly 200 is removed and the locking nut 130 tightened as described heretofore. Any extra length of the guide rod 120 is then cut flush with the locking nut 130.

Figure 21 illustrates an embodiment of the guide rod 300 for use with fastening devices without the tapered internal surface 166. As with the threaded portion 122 of the guide rod 120 the threaded section 302 of the guide rod 300 is locked into position for rotation by the guide rod holder clamp 250.

Figure 22 shows a fastening device for medical use, in this example a bone screw 150 such as disclosed in U.S. Patent 10,136,930 B2, into which the driver 200 inserted in order to insert or remove the screw 150. The screw 150 is composed of three sections; proximal segment 154 for driver attachment, a central flexible segment 152 and a distal segment 156 having distal screw threads 157.

The use of the embodiment illustrated in Figures 21 and 22 in conjunction with the tool assembly 100 and handle sub-assembly 200 are the same as the fastening device 150 with the exclusion of the distal tapered surface 165.

To use the disclosed handle sub-assembly 200 to remove a fastening device 150, the guide rod locking nut 130 is removed and the fastener coupling mechanism 230 reengaged with the proximal segment 154 of the fastening device 150. The rotation of the handle sub-assembly 200 is reversed and the fastening device is removed simultaneously from both the distal end 162 and the proximal end 164.

## Claims

1. A tool assembly for manipulating a fastening device (150) of at least one element, said tool assembly comprising:
a. a flexible guide rod (120), said flexible guide rod (120) comprising:
i. a proximal threaded section (121), said proximal threaded section (121) having a proximal end;
ii. a mid-section (123);
iii. a distal section (125), said distal section (125) having a tapered distal end (128);
iv. a length, said length extending from said proximal end of said proximal threaded section (121) to said tapered distal end (128) and having at least one cross-section configuration;
b. a fastening device (150) having a distal segment (156) with an open tapered distal end (162); a proximal segment (154) with an open proximal end (164); a central flexible segment (152) extending from said distal segment (156) to said proximal segment (154); and a fastener channel (167) extending through said fastening device (150) from said tapered distal end (162) to a proximal receptacle (155) within said proximal end (164), wherein said fastener channel (167) has at least one cross-section configuration and is configured to receive said flexible guide rod (120); and
c. a handle sub-assembly (200); said handle sub-assembly (200) comprising:
i. a handle (210) having:
1. a first end,
2. a second end;
3. a top side having a handle shank receiving area (211);
4. a bottom side open to said handle shank receiving area (211);
5. a clamp orifice (212) extending into said handle (210) from said first end past a mid-point of said handle (210);
ii. a coupling mechanism (230) configured to receive said proximal receptacle (155);
iii. a guide rod channel (228) extending through said coupling mechanism (230) configured to receive said proximal end of said flexible guide rod (120);
iv. a handle shank (220), said handle shank (220) extending from said bottom side of said handle (210) to connect said handle (210) and a proximal end of said coupling mechanism (230);
v. a guide rod shank passage (222) extending from said guide rod channel (228) and through said handle shank (220) and said handle (210);
vi. a guide rod holder clamp (250) having a securing member receiving area and being dimensioned to be received within said clamp orifice (212);
vii. a guide rod holder clamp securing member (255);
**characterized in that** said handle sub-assembly (200) further comprising:
- a guide rod hole (254) dimensioned along said guide rod holder clamp (250) to align with said guide rod shank passage (222) and dimensioned to receive said flexible guide rod (120); and
- a guide rod holder assembly (238), said guide rod holder assembly (238) comprising:
1. a guide rod holder (240); and
2. a holder shaft (244), said holder shaft (244) having:
a. a guide rod mating passage (247) extending through a distal portion of said holder shaft (244); and
b. a guide rod shaft passage (245) extending through a proximal portion of said holder shaft (244) and said guide rod holder (240); said guide rod shaft passage (245) being dimensioned to receive said flexible guide rod (120);
wherein one of said at least one cross-section configuration of said flexible guide rod is configured to interact with at least one of said at least one cross-section configuration of said fastening device channel (167) to enable said fastening device to be manipulated within said at least one element by initiating rotation of said fastening device at said tapered distal end.

2. The tool assembly of claim 1 wherein said distal section (125) of said flexible guide rod (120) is tapered, said distal section (125) having a proximal end and a distal end (128), said proximal end adjacent said mid-section (123) of said flexible guide rod (120).

3. The tool assembly of claim 2 wherein said distal end (128) of said distal section (125) has an outward taper, wherein said distal tapered section (125) is dimensioned to engage with a tapered internal surface (166) of said distal end (156) of said fastening device (150).

4. The tool assembly of claim 3 wherein said tapered internal surface (166) of said fastening device (150) is dimensioned to prevent said distal end (128) of said distal section (125) from passing through said distal end (162) of said fastening device (150).

5. The tool assembly of claim 1 wherein said guide rod mating passage (247) has a cross-section complimentary to said guide rod (120) to enable rotation of said guide rod (120) by said guide rod holder (240).

6. The tool assembly of claim 1 wherein said midsection (152) of said fastening device (150) is flexible to enable connection of more than one of said at least one element having non-linear connection channels.

7. The tool assembly of claim 1 wherein said fastening device (150) further comprises threads (157) along said distal segment (156), threads (158) along said proximal segment (154), and at least one sinuous slot (160) along said midsection (152) to allow flexibility of said fastening device.

8. The tool assembly of claim 1 wherein said flexible guide rod (120) is dimensioned to extend through said proximal receptacle (155) of said fastening device (150) and engage with said guide rod mating passage (247).

9. The tool assembly of claim 1 wherein said fastener coupling mechanism (230) has a cross section complementary to a cross section of a proximal mating surface (153) of said open proximal end (164) of said fastening device (150).

10. The tool assembly of claim 1 wherein said at least one cross-section of said fastening device channel (167) is a cross-section of a distal alignment surface (159) within said fastening device (150) and is complimentary to said predetermined configuration of said guide rod (120).

11. The tool assembly of claim 1 wherein said securing member receiving area of said guide rod holder clamp (250) is interior threads (252) and said tightening member is a guide rod holder clamp screw (255) having clamp screw threads (257) to interact with said interior threads (252).

12. The tool assembly of claim 1 wherein said guide rod (120) has a cross section less than said holder shaft passage (245) of said guide rod shaft (244) or less than an interior cross section of said distal segment (156) to create a gap (148) for torque transmission while enabling manipulation of said fastening device (150).

13. The tool assembly of claim 1 further comprising a locking nut having interior threads (136) dimensioned to be received on said guide rod (120) proximal threaded section (121).

14. The tool assembly of claim 13 wherein said locking nut further comprises a body (130) dimensioned to be received within said proximal receptacle (155) of said fastening device (150).

15. A method of manipulating a fastening device using a tool assembly according to claim 1 comprising the steps of:
a. Inserting a guide rod holder clamp (250) having a guide rod hole (254) and a threaded receiving area into a clamp orifice (212) within a handle (210) of a handle sub-assembly (200);
b. Aligning said guide rod hole (254) with a holder shaft receiving area (211) within handle (210);
c. extending a holder shaft (244) of a guide rod assembly (238) through said holder shaft receiving area (211) and into a handle shank (220) extending from a bottom side of said handle (210) to a coupling mechanism (230);
d. Inserting a proximal threaded section (121) of a flexible guide rod (120), having at least one cross-sectional configuration, into an open tapered distal end (162) of a fastening device (150) having a fastener channel (167) with a cross-sectional configuration;
e. extending said flexible guide rod threaded section (121) through a distal segment (156) and a central flexible segment (152) to extend beyond a proximal segment (154) of said fastening device (150);
f. Continuing to insert said flexible guide rod (120) through said fastener channel (167) until a tapered distal end (128) of a distal section (125) of said guide rod (120) contacts said open tapered distal end (162) of said fastening device (150);
g. inserting said threaded section (121) of said flexible guide rod (120) into a guide rod channel (228) within said coupling mechanism (230);
h. inserting said coupling mechanism (230) into a proximal receptacle (155) within a proximal segment (154) of said fastening device (150);
i. aligning a cross-section configuration of said proximal receptacle (155) with a cross section configuration of said coupling mechanism (230);
j. aligning a cross section configuration of said threaded section (121) with a cross section configuration of said guide rod shaft passage (245) of holder shaft (244);
k. preventing movement of said holder shaft (244) by pulling said guide rod hole 254 against said holder shaft 244 by inserting a threaded securing member into said guide rod holder clamp (250);
l. manipulating said fastening device (150) by rotating said guide rod holder (240) within said guide rod assembly (238) to engage tapered distal section 125 of guide rod 120 with fastener channel 167 at said distal segment (156) of said fastening device (150) to initiate rotation of said of said fastening device (150) at said tapered distal end (128);
m. removing said handle sub-assembly (200) when said fastening device (150) is positioned in an object not being a bone;
n. threading a nut (130) onto said threaded section (121);
o. pulling said tapered distal section (125) into said tapered distal end (162) of said fastening device (150) by tightening said nut (130); and
p. removing excess threaded section (121).

## Patentansprüche

1. Eine Werkzeuganordnung zum Manipulieren einer Befestigungsvorrichtung (150) von mindestens einem Element, wobei die Werkzeuganordnung Folgendes umfasst:
A. eine flexible Führungsstange (120), wobei die flexible Führungsstange (120) umfasst:
i. einen proximalen Gewindeabschnitt (121), wobei der proximale Gewindeabschnitt (121) ein proximales Ende aufweist;
ii. ein Mittelteil (123);
iii. einen distalen Abschnitt (125), wobei der distale Abschnitt (125) ein konisches distales Ende (128) aufweist;
iv. eine Länge, wobei sich die Länge vom proximalen Ende des proximalen Gewindeabschnitts (121) bis zum konischen distalen Ende (128) erstreckt und mindestens eine Querschnittskonfiguration aufweist;
B. eine Befestigungsvorrichtung (150) mit einem distalen Segment (156) mit einem offenen, konischen distalen Ende (162); ein proximales Segment (154) mit einem offenen proximalen Ende (164); ein zentrales flexibles Segment (152), das sich vom distalen Segment (156) zum proximalen Segment (154) erstreckt; und einen Befestigungskanal (167), der sich durch die Befestigungsvorrichtung (150) vom konischen distalen Ende (162) zu einer proximalen Aufnahme (155) innerhalb des proximalen Endes (164) erstreckt, wobei der Befestigungskanal (167) mindestens eine Querschnittskonfiguration aufweist und zur Aufnahme der flexiblen Führungsstange (120) konfiguriert ist; Und
C. eine Griffunterbaugruppe (200); wobei die Griffunterbaugruppe (200) umfasst:
i. einen Griff (210) mit:
1. ein erstes Ende,
2. ein zweites Ende;
3. eine Oberseite mit einem Griffschaftaufnahmebereich (211);
4. eine Unterseite, die zum Griffschaftaufnahmebereich (211) hin offen ist;
5. eine Klemmöffnung (212), die sich vom ersten Ende über einen Mittelpunkt des Griffs (210) hinaus in den Griff (210) erstreckt;
ii. einen Kopplungsmechanismus (230), der so konfiguriert ist, dass er die proximale Aufnahme (155) aufnimmt;
iii. einen Führungsstangenkanal (228), der sich durch den Kopplungsmechanismus (230) erstreckt und so konfiguriert ist, dass er das proximale Ende der flexiblen Führungsstange (120) aufnimmt;
iv. einen Griffschaft (220), wobei sich der Griffschaft (220) von der Unterseite des Griffs (210) erstreckt, um den Griff (210) und ein proximales Ende des Kopplungsmechanismus (230) zu verbinden;
v. einen Führungsstangenschaftdurchgang (222), der sich von dem Führungsstangenkanal (228) und durch den Griffschaft (220) und den Griff (210) erstreckt;
vi. eine Führungsstangenhalterklemme (250), die einen Aufnahmebereich für ein Befestigungselement aufweist und so dimensioniert ist, dass sie in der Klemmenöffnung (212) aufgenommen werden kann;
vii. ein Sicherungselement (255) für die Klemme des Führungsstangenhalters;
**dadurch gekennzeichnet, dass** die Griffunterbaugruppe (200) weiterhin umfasst:
- ein Führungsstangenloch (254), das entlang der Führungsstangenhalterklemme (250) dimensioniert ist, um mit dem Führungsstangenschaftdurchgang (222) ausgerichtet zu sein, und dimensioniert ist, um die flexible Führungsstange (120) aufzunehmen; Und
- eine Führungsstangenhalterbaugruppe (238), wobei die Führungsstangenhalterbaugruppe (238) umfasst:
1. ein Führungsstangenhalter (240); Und
2. eine Halterwelle (244), wobei die Halterwelle (244) aufweist:
A. einen Führungsstangenpassdurchgang (247), der sich durch einen distalen Abschnitt des Halterschafts (244) erstreckt; Und
B. einen Führungsstangenschaftdurchgang (245), der sich durch einen proximalen Abschnitt des Halterschafts (244) und des Führungsstangenhalters (240) erstreckt; wobei der Führungsstangenschaftdurchgang (245) so dimensioniert ist, dass er die flexible Führungsstange (120) aufnimmt;
wobei eine der mindestens einen Querschnittskonfigurationen der flexiblen Führungsstange so konfiguriert ist, dass sie mit mindestens einer der mindestens einen Querschnittskonfigurationen des Befestigungsvorrichtungskanals (167) zusammenwirkt, um zu ermöglichen, dass die Befestigungsvorrichtung manipuliert wird in das mindestens eine Element durch Auslösen der Drehung der Befestigungsvorrichtung am konischen distalen Ende.

2. Werkzeuganordnung nach Anspruch 1, wobei der distale Abschnitt (125) der flexiblen Führungsstange (120) konisch ist, wobei der distale Abschnitt (125) ein proximales Ende und ein distales Ende (128) aufweist, wobei das proximale Ende an das Mittelteil (123) der flexiblen Führungsstange (120) angrenzt.

3. Werkzeuganordnung nach Anspruch 2, wobei das distale Ende (128) des distalen Abschnitts (125) eine nach außen gerichtete Konizität aufweist, wobei der distale konischen Abschnitt (125) so dimensioniert ist, dass er mit einer konischen Innenfläche (166) des distalen Endes in Eingriff kommt Ende (156) der Befestigungsvorrichtung (150).

4. Werkzeuganordnung nach Anspruch 3, wobei die konische Innenfläche (166) der Befestigungsvorrichtung (150) so dimensioniert ist, dass sie verhindert, dass das distale Ende (128) des distalen Abschnitts (125) durch das distale Ende (162) der Befestigungsvorrichtung (150) hindurchtritt.

5. Werkzeuganordnung nach Anspruch 1, wobei der Führungsstangenpassdurchgang (247) einen Querschnitt aufweist, der komplementär zu der Führungsstange (120) ist, um eine Drehung der Führungsstange (120) durch den Führungsstangenhalter (240) zu ermöglichen.

6. Werkzeuganordnung nach Anspruch 1, wobei der Mittelabschnitt (152) der Befestigungsvorrichtung (150) flexibel ist, um die Verbindung von mehr als einem der mindestens einen Elemente mit nichtlinearen Verbindungskanälen zu ermöglichen.

7. Werkzeuganordnung nach Anspruch 1, wobei die Befestigungsvorrichtung (150) außerdem Gewinde (157) entlang des distalen Segments (156), Gewinde (158) entlang des proximalen Segments (154) und mindestens einen gewundenen Schlitz (160) entlang des Mittelabschnitts (152) umfasst, um Flexibilität der Befestigungsvorrichtung zu ermöglichen.

8. Werkzeuganordnung nach Anspruch 1, wobei die flexible Führungsstange (120) so dimensioniert ist, dass sie sich durch die proximale Aufnahme (155) der Befestigungsvorrichtung (150) erstreckt und in den passenden Führungsstangendurchgang (247) eingreift.

9. Werkzeuganordnung nach Anspruch 1, wobei der Verbindungsmechanismus (230) für das Befestigungselement einen Querschnitt aufweist, der komplementär zu einem Querschnitt einer proximalen Passfläche (153) des offenen proximalen Endes (164) der Befestigungsvorrichtung (150) ist.

10. Werkzeuganordnung nach Anspruch 1, wobei der mindestens eine Querschnitt des Befestigungsvorrichtungskanals (167) ein Querschnitt einer distalen Ausrichtungsfläche (159) innerhalb der Befestigungsvorrichtung (150) ist und zu dem vorgegebenen Querschnitt komplementär ist zu der Konfiguration der Führungsstange (120).

11. Werkzeuganordnung nach Anspruch 1, wobei der Aufnahmebereich für das Sicherungselement der Führungsstangenhalterklemme (250) ein Innengewinde (252) ist und das Spannelement eine Führungsstangenhalterklemmschraube (255) mit Klemmschraubengewinde (257) ist, um mit den Innengewinden (252) zusammenzuwirken.

12. Werkzeuganordnung nach Anspruch 1, wobei die Führungsstange (120) einen Querschnitt aufweist, der kleiner als der Halterschaftdurchgang (245) des Führungsstangenschafts (244) oder kleiner als ein Innenquerschnitt des distalen Segments (156) ist, um einen Spalt (148) zur Drehmomentübertragung zu schaffen und gleichzeitig eine Manipulation der Befestigungsvorrichtung (150) zu ermöglichen.

13. Werkzeuganordnung nach Anspruch 1, ferner umfassend eine Sicherungsmutter mit Innengewinde (136), die so bemessen ist, dass sie auf dem proximalen Gewindeabschnitt (121) der Führungsstange (120) aufgenommen werden kann.

14. Werkzeuganordnung nach Anspruch 13, wobei die Sicherungsmutter außerdem einen Körper (130) umfasst, der so dimensioniert ist, dass er in der proximalen Aufnahme (155) der Befestigungsvorrichtung (150) aufgenommen werden kann.

15. Verfahren zur Handhabung einer Befestigungsvorrichtung unter Verwendung einer Werkzeuganordnung nach Anspruch 1, umfassend die Schritte:
A. Einsetzen einer Führungsstangenhalterklemme (250) mit einem Führungsstangenloch (254) und einem Gewindeaufnahmebereich in eine Klemmenöffnung (212) innerhalb eines Griffs (210) einer Griffunterbaugruppe (200);
B. Ausrichten des Führungsstangenlochs (254) mit einem Aufnahmebereich (211) für den Halterschaft im Griff (210);
C. Erweitern eines Halterschafts (244) einer Führungsstangenanordnung (238) durch den Halterschaftaufnahmebereich (211) und in einen Griffschaft (220), der sich von einer Unterseite des Griffs (210) zu einem Kopplungsmechanismus (230) erstreckt;
D. Einführen eines proximalen Gewindeabschnitts (121) eines flexiblen Führungsstabs (120) mit mindestens einer Querschnittskonfiguration in ein offenes, konisches distales Ende (162) einer Befestigungsvorrichtung (150), die einen Befestigungskanal (167) aufweist mit eine Querschnittskonfiguration;
E. Erweitern des flexiblen Führungsstabgewindeabschnitts (121) durch ein distales Segment (156) und ein zentrales flexibles Segment (152), um sich über ein proximales Segment (154) der Befestigungsvorrichtung (150) hinaus zu erstrecken;
F. Weiterführendes Einführen des flexiblen Führungsstabs (120) durch den Befestigungskanal (167), bis ein konisches distales Ende (128) eines distalen Abschnitts (125) des Führungsstabs (120) das offene, konische distale Ende (162) berührt Befestigungsvorrichtung (150);
G. Einführen des Gewindeabschnitts (121) der flexiblen Führungsstange (120) in einen Führungsstangenkanal (228) innerhalb des Kopplungsmechanismus (230);
H. Einsetzen des Kopplungsmechanismus (230) in eine proximale Aufnahme (155) innerhalb eines proximalen Segments (154) der Befestigungsvorrichtung (150);
I. Ausrichten einer Querschnittskonfiguration der proximalen Aufnahme (155) mit einer Querschnittskonfiguration des Kopplungsmechanismus (230);
J. Ausrichten einer Querschnittskonfiguration des Gewindeabschnitts (121) mit einer Querschnittskonfiguration des Führungsstangenschaftdurchgangs (245) des Halterschafts (244);
K. Verhindern einer Bewegung des Halterschafts (244), indem das Führungsstangenloch (254) gegen den Halterschaft (244) gezogen wird, indem ein Befestigungselement mit Gewinde in die Führungsstangenhalterklemme (250) eingeführt wird;
L. Manipulieren der Befestigungsvorrichtung (150) durch Drehen des Führungsstangenhalters (240) innerhalb der Führungsstangenanordnung (238), um den konischen distalen Abschnitt (125) der Führungsstange (120) mit dem Befestigungskanal (167) am distalen Segment (156) der Befestigungsvorrichtung in Eingriff zu bringen (150), um die Drehung der Befestigungsvorrichtung (150) am konischen distalen Ende (128) einzuleiten;
M. Entfernen der Griffunterbaugruppe (200), wenn die Befestigungsvorrichtung (150) in einem Objekt positioniert ist, das kein Knochen ist;
N. Aufschrauben einer Mutter (130) auf den Gewindeabschnitt (121);
O. Ziehen des konischen distalen Abschnitts (125) in das konische distale Ende (162) der Befestigungsvorrichtung (150) durch Anziehen der Mutter (130); Und
P. Entfernen des überschüssigen Gewindeabschnitts (121).

## Revendications

1. Ensemble outil pour manipuler un dispositif de fixation (150) d'au moins un élément, ledit ensemble outil comprenant :
a. une tige de guidage flexible (120), ladite tige de guidage flexible (120) comprenant:
i. une section filetée proximale (121), ladite section filetée proximale (121) ayant une extrémité proximale ;
ii. une section médiane (123);
iii. une section distale (125), ladite section distale (125) ayant une extrémité distale effilée (128);
iv. une longueur, ladite longueur s'étendant depuis ladite extrémité proximale de ladite section filetée proximale (121) jusqu'à ladite extrémité distale effilée (128) et ayant au moins une configuration de section transversale;
b. un dispositif de fixation (150) ayant un segment distal (156) avec une extrémité distale effilée ouverte (162); un segment proximal (154) avec une extrémité proximale ouverte (164); un segment flexible central (152) s'étendant dudit segment distal (156) audit segment proximal (154); et un canal de fixation (167) s'étendant à travers ledit dispositif de fixation (150) depuis ladite extrémité distale effilée (162) jusqu'à un réceptacle proximal (155) à l'intérieur de ladite extrémité proximale (164), lequel ledit canal de fixation (167) ayant au moins une configuration de section transversale et est configurée pour recevoir ladite tige de guidage flexible (120); et
c. un sous-ensemble de poignée (200); ledit sous-ensemble de poignée (200) comprenant:
i. une poignée (210) comportant:
1. une première fin ;
2. une seconde extrémité;
3. un côté supérieur ayant une zone de réception de tige de poignée (211);
4. un côté inférieur ouvert sur ladite zone de réception de tige de poignée (211);
5. un orifice de serrage (212) s'étendant dans ladite poignée (210) depuis ladite première extrémité au-delà d'un point médian de ladite poignée (210);
ii. un mécanisme de couplage (230) configuré pour recevoir ledit réceptacle proximal (155);
iii. un canal de tige de guidage (228) s'étendant à travers ledit mécanisme de couplage (230) configuré pour recevoir ladite extrémité proximale de ladite tige de guidage flexible (120);
iv. une tige de poignée (220), ladite tige de poignée (220) s'étendant depuis ledit côté inférieur de ladite poignée (210) pour relier ladite poignée (210) et une extrémité proximale dudit mécanisme de couplage (230);
v. un passage de tige de tige de guidage (222) s'étendant depuis ledit canal de tige de guidage (228) et à travers ladite tige de poignée (220) et ladite poignée (210);
vi. une pince de support de tige de guidage (250) ayant une zone de réception d'élément de fixation et étant dimensionnée pour être reçue à l'intérieur dudit orifice de serrage (212);
vii. un élément de fixation de pince de support de tige de guidage (255);
**caractérisé en ce que** ledit sous-ensemble de poignée (200) comprend en outre:
- un trou de tige de guidage (254) dimensionné le long de ladite pince de support de tige de guidage (250) pour s'aligner avec ledit passage de tige de tige de guidage (222) et dimensionné pour recevoir ladite tige de guidage flexible (120); et
- un ensemble porte-tige de guidage (238), ledit ensemble porte-tige de guidage (238) comprenant:
1. un support de tige de guidage (240); et
2. un arbre de support (244), ledit arbre de support (244) comportant:
a. un passage d'accouplement de tige de guidage (247) s'étendant à travers une partie distale dudit arbre de support (244); et
b. un passage d'arbre de tige de guidage (245) s'étendant à travers une partie proximale dudit arbre de support (244) et dudit support de tige de guidage (240); ledit passage d'arbre de tige de guidage (245) étant dimensionné pour recevoir ladite tige de guidage flexible (120);
dans lequel l'une desdites au moins une configuration de section transversale de ladite tige de guidage flexible est configurée pour interagir avec au moins une de ladite au moins une configuration de section transversale dudit canal de dispositif de fixation (167) pour permettre audit dispositif de fixation d'être manipulé à l'intérieur ledit au moins un élément en initiant la rotation dudit dispositif de fixation au niveau de ladite extrémité distale effilée.

2. Ensemble d'outils selon la revendication 1, dans lequel ladite section distale (125) de ladite tige de guidage flexible (120) est effilée, ladite section distale (125) ayant une extrémité proximale et une extrémité distale (128), ladite extrémité proximale adjacente audit section médiane (123) de ladite tige de guidage flexible (120).

3. Ensemble d'outils selon la revendication 2, dans lequel ladite extrémité distale (128) de ladite section distale (125) a une conicité vers l'extérieur, dans lequel ladite section distale effilée (125) est dimensionnée pour s'engager avec une surface interne conique (166) de ladite distale extrémité (156) dudit dispositif de fixation (150).

4. Ensemble d'outils selon la revendication 3, dans lequel ladite surface interne effilée (166) dudit dispositif de fixation (150) est dimensionnée pour empêcher ladite extrémité distale (128) de ladite section distale (125) de passer à travers ladite extrémité distale (162) de ledit dispositif de fixation (150).

5. Ensemble d'outils selon la revendication 1, dans lequel ledit passage d'accouplement de tige de guidage (247) a une section transversale complémentaire à ladite tige de guidage (120) pour permettre la rotation de ladite tige de guidage (120) par ledit support de tige de guidage (240).

6. Ensemble d'outils selon la revendication 1, dans lequel ladite section médiane (152) dudit dispositif de fixation (150) est flexible pour permettre la connexion de plus d'un dudit au moins un élément ayant des canaux de connexion non linéaires.

7. Ensemble d'outils selon la revendication 1, dans lequel ledit dispositif de fixation (150) comprend en outre des filetages (157) le long dudit segment distal (156), des filetages (158) le long dudit segment proximal (154), et au moins une fente sinueuse (160) le long de ladite section médiane (152) pour permettre la flexibilité dudit dispositif de fixation.

8. Ensemble d'outils selon la revendication 1, dans lequel ladite tige de guidage flexible (120) est dimensionnée pour s'étendre à travers ledit réceptacle proximal (155) dudit dispositif de fixation (150) et s'engager avec ledit passage d'accouplement de tige de guidage (247).

9. Ensemble d'outils selon la revendication 1, dans lequel ledit mécanisme de couplage de fixation (230) a une section transversale complémentaire à une section transversale d'une surface de contact proximale (153) de ladite extrémité proximale ouverte (164) dudit dispositif de fixation (150).

10. Ensemble d'outils selon la revendication 1, dans lequel ladite au moins une section transversale dudit canal du dispositif de fixation (167) est une section transversale d'une surface d'alignement distale (159) à l'intérieur dudit dispositif de fixation (150) et est complémentaire de prédéterminé configuration de ladite tige de guidage (120).

11. Ensemble d'outils selon la revendication 1, dans lequel ladite zone de réception de l'élément de fixation de ladite pince de support de tige de guidage (250) est constituée de filetages intérieurs (252) et ledit élément de serrage est une vis de serrage de support de tige de guidage (255) ayant des filetages de vis de serrage (257), pour interagir avec lesdits fils intérieurs (252).

12. Ensemble d'outils selon la revendication 1, dans lequel ladite tige de guidage (120) a une section transversale inférieure audit passage d'arbre de support (245) dudit arbre de tige de guidage (244) ou inférieure à une section transversale intérieure dudit segment distal (156), pour créer un espace (148) pour la transmission du couple tout en permettant la manipulation dudit dispositif de fixation (150).

13. Ensemble d'outils selon la revendication 1, comprenant en outre un écrou de blocage ayant des filetages intérieurs (136) dimensionnés pour être reçus sur ladite tige de guidage (120) sur la section filetée proximale (121).

14. Ensemble d'outils selon la revendication 13, dans lequel ledit écrou de blocage comprend en outre un corps (130) dimensionné pour être reçu à l'intérieur dudit réceptacle proximal (155) dudit dispositif de fixation (150).

15. Procédé de manipulation d'un dispositif de fixation à l'aide d'un ensemble outil selon l'une des revendications précédentes comprenant les étapes de:
a. insérer une pince de support de tige de guidage (250) ayant un trou de tige de guidage (254) et une zone de réception filetée dans un orifice de serrage (212) à l'intérieur d'une poignée (210) d'un sous-ensemble de poignée (200);
b. aligner ledit trou de tige de guidage (254) avec une zone de réception d'arbre de support (211) à l'intérieur de la poignée (210);
c. étendre un arbre de support (244) d'un ensemble de tige de guidage (238) à travers ladite zone de réception d'arbre de support (211) et dans une tige de poignée (220) s'étendant depuis un côté inférieur de ladite poignée (210) jusqu'à un mécanisme d'accouplement (230);
d. insertion d'une section filetée proximale (121) d'une tige de guidage flexible (120), ayant au moins une configuration en coupe transversale, dans une extrémité distale conique ouverte (162) d'un dispositif de fixation (150) ayant un canal de fixation (167) avec une configuration transversale;
e. étendre ladite section filetée de tige de guidage flexible (121) à travers un segment distal (156) et un segment flexible central (152) pour s'étendre au-delà d'un segment proximal (154) dudit dispositif de fixation (150);
f. continuer à insérer ladite tige de guidage flexible (120) à travers ledit canal de fixation (167) jusqu'à ce qu'une extrémité distale effilée (128) d'une section distale (125) de ladite tige de guidage (120) entre en contact avec ladite extrémité distale effilée ouverte (162) dudit dispositif de fixation (150);
g. insérer ladite section filetée (121) de ladite tige de guidage flexible (120) dans un canal de tige de guidage (228) à l'intérieur dudit mécanisme de couplage (230);
h. insérer ledit mécanisme de couplage (230) dans un réceptacle proximal (155) à l'intérieur d'un segment proximal (154) dudit dispositif de fixation (150);
i. aligner une configuration de section transversale dudit réceptacle proximal (155) avec une configuration de section transversale dudit mécanisme de couplage (230);
j. aligner une configuration de section transversale de ladite section filetée (121) avec une configuration de section transversale dudit passage d'arbre de tige de guidage (245) de l'arbre de support (244);
k. empêcher le mouvement dudit arbre de support (244) en tirant ledit trou de tige de guidage 254 contre ledit arbre de support 244 en insérant un élément de fixation fileté dans ladite pince de support de tige de guidage (250);
l. manipuler ledit dispositif de fixation (150) en faisant tourner ledit support de tige de guidage (240) à l'intérieur dudit ensemble de tige de guidage (238) pour engager la section distale effilée 125 de la tige de guidage 120 avec le canal de fixation 167 au niveau dudit segment distal (156) dudit dispositif de fixation ( 150) pour initier la rotation dudit dispositif de fixation (150) au niveau de ladite extrémité distale effilée (128);
m. retirer ledit sous-ensemble de poignée (200) lorsque ledit dispositif de fixation (150) est positionné dans un objet qui n'est pas un os;
n. visser un écrou (130) sur ladite section filetée (121);
o. tirer ladite section distale effilée (125) dans ladite extrémité distale effilée (162) dudit dispositif de fixation (150) en serrant ledit écrou (130); et
p. retirer l'excédent de section filetée (121).
